# EUROPEAN PATENT SPECIFICATION

(11) **EP 2 465 854 B1**
(45) Date of publication and mention of the grant of the patent: **13.11.2013**
(21) Application number: 11192943.6
(22) Date of filing: 12.12.2011
(51) Int. Cl.: C07D 251/60

(54) **Use of urea containing formaldehyde in a process for the production of melamine by the pyrolysis of urea and related process for the production of melamine**
Verwendung von Formaldehyd-Enthaltendem Harnstoff in einem Verfahren zur Herstellung von Melamin durch Pyrolyse von Harnstoff und verwandtes Verfahren zur Herstellung von Melamin
Utilisation d'urée contenant du formaldéhyde dans un procédé de préparation de la mélamine par pyrolyse d'urée et le procédé apparenté de préparation de la mélamine

(30) Priority: 15.12.2010 IT IT20102286
(43) Date of publication of application: 20.06.2012
(73) Proprietor: EUROTECNICA MELAMINE, Luxembourg Zweigniederlassung in Ittigen, 3063 Ittigen (CH)
(72) Inventor: Santucci, Roberto, I-21050 GORLA MAGGIORE - VARESE, Italy (IT)
(74) Representative: De Gregori, Antonella

(56) References cited:
- William Cameron: "Cyanamides" In: "Kirk-Othmer Encyclopedia of Chemical Technology", 17 September 2010 (2010-09-17), John Wiley & Sons, Inc., XP55002324, pages 1-15, * page 10, paragraph 4.2 - page 12 *

## Description

The present invention refers to the use of urea containing formaldehyde in a process for the production of melamine by the pyrolysis of urea and to the related process for the production of melamine.

More in particular, the present invention refers to the use of urea containing formaldehyde as raw material fed to the pyrolysis reactor, in a process for the production of melamine which successively provides the purification of melamine produced in the pyrolysis reactor and its separation as end-product.

It is known that the transformation of molten urea into melamine is described by the following global reaction (1) : 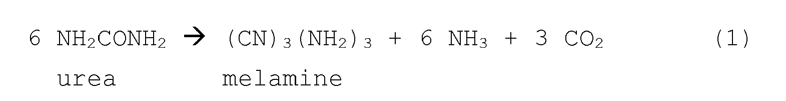 according to which for each kilogram of melamine 1,86 kilograms of NH₃ and CO₂ are formed, globally called off-gas (see for example the chapter on Cyanamides, paragraph "4. Melamine", in Kirk-Othmer Encyclopedia of Chemical Technology).

The processes for the transformation of urea into melamine may substantially be distinguished into two groups and specifically the processes carrying out the pyrolysis of urea at high pressure and the processes carrying out the pyrolysis of urea at low pressure.

Applicants surprisingly found that when the process for the production of melamine by the pyrolysis of urea is carried out starting from urea containing formaldehyde, the end-products obtained from melamine thus achieved present characteristics of particular interest.

It is therefore an objective of the present invention the use of urea containing formaldehyde in a process for the production of melamine by the pyrolysis of urea.

A further objective of the present invention is the process for the production of melamine by the pyrolysis of urea starting from urea containing formaldehyde.

In particular the amount of formaldehyde present in the urea fed to the pyrolysis reactor ranges from 10 to 5000 ppm by weight, preferably from 50 to 2000 ppm by weight, and even more preferably from 100 to 500 ppm by weight.

The formaldehyde may be present in the urea following to the process of production of the urea itself, or it may be suitably added to the urea; a combined case may also exist, wherein the formaldehyde is added to a urea already provided therewith.

Therefore, in the present application the expression "urea containing formaldehyde" means both the urea as end-product, which already contains formaldehyde as a result of its own productive process, and the urea as end-product which does not contain formaldehyde, the latter being successively added, and also urea as end-product already containing formaldehyde, the latter being further added of formaldehyde.

The urea used for the production of melamine may come from one or more urea plants, both as an internal stream of the urea plants themselves (that is withdrawn between two steps of the urea process and then dehydrated), and as an end-product urea.

When the urea used for the production of melamine comes from an internal stream of the urea plant, said stream may contain formaldehyde. This is an advantage with respect to the current state of the art, according to which only streams containing urea without formaldehyde are sent to the melamine plants.

When the urea used for the production of melamine is an end-product urea, such a urea may be of any type ("grade"), containing formaldehyde or not.

For example, such a urea may be of the type for fertilizers ("fertiliser grade", containing formaldehyde) or of the technical type ("technical grade", not containing formaldehyde). It is also possible to use mixtures of urea of different types. This a further advantage of the present invention with respect to the state of the art, according to which for the melamine plants are used only types of urea not containing formaldehyde, usually "technical grade" urea. That kind of urea is in fact available on the market in lower quantities and is more expensive than the "fertiliser grade" urea, in that it is usually produced on demand and campaigns, upon renewal of the plant. This greater flexibility in the use of the raw material provides more freedom in the localization of the melamine plants, which may be supplied with urea by the local market.

Moreover, the formaldehyde added to the urea may be admixed as such or as compound or as mixture containing formaldehyde, such as for example formurea (aqueous solution at high concentration of urea and formaldehyde, for example 23 and 57 % by weight respectively for the so-called formurea 80).

As previously observed, the use of urea containing formaldehyde as raw material fed to a process for the production of melamine allows obtaining a melamine from which end-products derive that present characteristics of particular interest.

In fact, the end-products obtained starting from said melamine present particular characteristics of brightness (whichever is their colour) and of whiteness (if they are white), as well as greater resistance to aging, said characteristics being achievable with a lower consumption of dyestuffs and/or other additives, such as for example the titanium dioxide TiO₂.

Such an unexpected improvement of the characteristics of the product obtained starting from the melamine thus prepared is observed in each kind of end-product in which the surface aspect is important: laminates, moulding compounds, surface coatings, etc.

Moreover, the melamine obtained by the process of pyrolysis of the urea starting from urea containing formaldehyde according to the present invention, in some processes it may be visually identified, in that it is fluorescent if observed under ultraviolet light. Such an effect may be observed over a certain amount of formaldehyde in the urea.

As mentioned, it is also an objective of the present invention the process for the production of melamine by the pyrolysis of urea starting from urea containing formaldehyde. Such a process for the production of melamine by the pyrolysis of urea may be a high-pressure process or a low-pressure process and is preferably a high-pressure process.

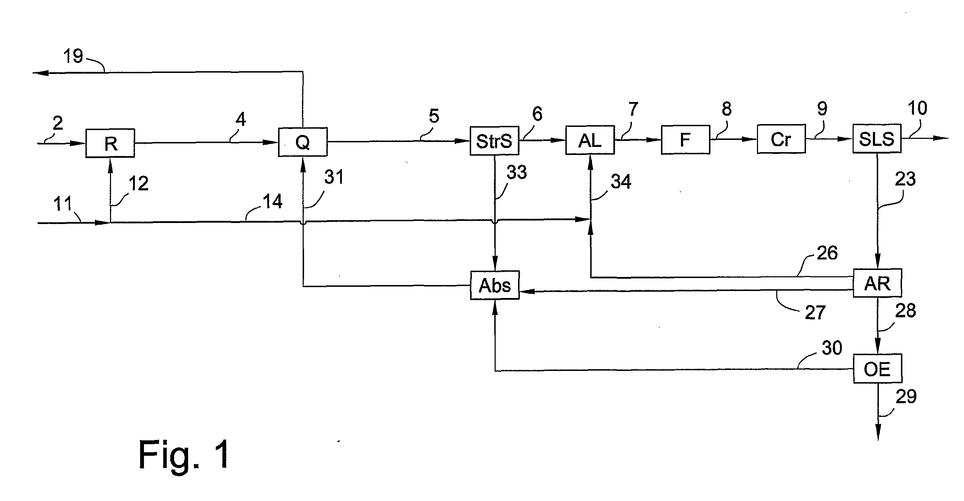

As a mere illustrative example, it is reported a high-pressure process which can be carried out starting from urea containing formaldehyde according to the present invention. One of the most spread industrial processes based on pyrolysis of urea at high-pressure is that described in the patent US 3,161,638 in the name of Allied. In this process the whole bi-phase effluent coming from the synthesis reactor of melamine is cooled and collected in an ammonia aqueous solution. To illustrate the process according to the above mentioned patent US 3,161,638, in figure 1 is reported a simplified block scheme of an embodiment of the above mentioned process.

According to the scheme of figure 1, the urea (stream 2) produced in an adjacent synthesis (not shown in figure 1) is sent in the liquid step, at a temperature of 135 - 145 °C, to a reaction section R consisting of a pyrolysis reactor wherein a proper heating system maintains the reacting system at a temperature of about 360 - 420 °C; the pressure is maintained at a value greater than 70 barrel. Preferably, along with the molten urea also gaseous anhydrous NH₃ (stream 12) is introduced in the reactor. The reactor is a one-single step reactor, and the reacting mass is maintained in strong circulation from the gases formed during the pyrolysis of urea.

In an embodiment according to the present invention, the urea (stream 2) produced in an adjacent synthesis plant contains formaldehyde.

All the reacted mass, composed of a bi-phase liquid/gas effluent (stream 4), is discharged in a section of quench Q in which, by contact with an ammonia aqueous solution (stream 31), its temperature is decreased to about 160 °C. Under the aforesaid operative conditions all the melamine, the unreacted urea and the various impurities formed during the synthesis (for example oxy-amino-triazines OAT and polycondensates) pass in solution and are sent to the next treatment (stream 5), whereas the remaining gaseous phase, substantially consisting of NH₃ and CO₂ coming from the reactor and from the aqueous vapour of saturation, is separated and sent to the urea plant (stream 19 of wet off-gas), after being subjected to a possible treatment (not indicated in figure 1), such as for example the condensation by adsorption in aqueous solution.

The stream 5 contains a certain quantity of NH₃ and CO₂ dissolved, which are removed in the next stripping section with StrS vapour. The elimination of CO₂ is necessary to obtain a high purity of the melamine in the successive steps; the removal of NH₃ is not necessary, but is experienced due to the nature of the liquid/gas equilibriums in the system water - NH₃-CO0₂.

Two streams come from the StrS section: a gaseous stream (stream 33) comprising NH₃ and CO₂ removed from stream 5, and an aqueous stream comprising melamine and the remaining impurities (stream 6).

The stream 33 is sent to an absorption section Abs wherein it puts in contact with an aqueous stream 30, forming an aqueous stream 31 comprising CO₂ and NH₃ pulled out from the stream 5. The aqueous stream 31 is in turn sent to the quench section Q, wherein as above said it is used to cool and dissolve the stream 4 exiting from the reactor.

The aqueous stream exiting from the bottom of the StrS section (stream 6) contains residual CO₂ of about 0,3 - 0,5 % by mass, melamine of about 6 - 12 % by mass, impurities of OAT and polycondensates and also urea exited from the reactor and not hydrolyzed to NH₃ and CO₂ in the Q quench and the StrS stripping. Such a urea hydrolyzes in the sections of the plant downstream the StrS stripping, up to the section of deammoniation AR (see further), leading to the undesired production of new CO₂.

The polycondensates, due to their low solubility, must be removed before sending said stream 6 to the crystallization section Cr for recovery of melamine.

To eliminate the polycondensates, to the stream 6 is added NH₃ (stream 34) up to achieve 12 - 15 % by mass. The resulting stream stands at about 170 °C in an ammonolysis section AL, in an apparatus called "*ammonolyser*", wherein the polycondensates are almost totally eliminated and transformed for the most part in melamine.

The ammonia aqueous solution exiting from the AL section (stream 7) is sent to the filtration section of finishing F and then to the crystallization section Cr (stream 8), wherein the temperature is lowered to about 40 - 50 °C and the most part of melamine crystallizes. The high concentration of NH₃ and the low concentration of CO₂ in the crystallization allow maintaining the OAT in solution, whose solubility in the basic field is much increased when pH increases, and thus allow separating a product with a high purity (greater than 99,8 % by mass).

The ammonia aqueous suspension comprising the crystallized melamine exiting from the section Cr (stream 9) is sent to the section of solid/liquid separation SLS, wherein from the stream 9 are separated the crystallized melamine (stream 10) and a stream of mother liquors (stream 23) comprising the OAT, both those formed during the pyrolysis reaction, and those deriving from hydrolysis of melamine in the various apparatuses in which it remained standing in heat aqueous phase.

The stream 23 of mother liquors, wherein the residual melamine is present in the concentration of 0,8 - 1 % by mass, can not be directly recycled to the section of quench Q: such a direct recycle would imply an increase in the concentrations of OAT up to go beyond the concentration of saturation in crystallization, wherein they would precipitate along with the melamine, polluting the product. On the other hand the stream 23 of mother liquors can not be directly discharged in the environment due to environmental and economic problems connected to the strong content of NH₃ and organic compounds, among which melamine.

To avoid the above problems, the process according to the patent US 3,161,368 provides the deammoniation of mother liquors in a AR section in which three streams are separated by distillation: a stream of NH₃ substantially void of CO₂, to be recycled to the ammonolysis section AL (stream 26); a stream rich in CO₂, which is recycled to the Abs absorber (stream 27); an aqueous stream quite void of NH₃ and comprising almost exclusively melamine and OAT (stream 28).

The stream 28 of mother liquors deammoniated is sent to a section OE for the removal of the OAT and for obtaining an aqueous solution to recycle to the section of quench Q (stream 30). The OE section may be achieved in two different ways, both applied in the state of the art:
a) in one case, the OAT are crystallized by cooling and neutralization with CO₂, and then separated from the stream 28 by ultrafiltration. Said separation provides:
   - an aqueous stream 30 substantially void of OAT and rich in melamine, to recycle to the quench Q (through the Abs absorber) thus recovering also the melamine therein contained;
   - a stream rich in OAT in suspension to send to decomposition to recover the organic compounds in form of NH₃ and CO₂, obtaining an aqueous stream (stream 29) substantially void of impurities, which can be discharged or re-used in a suitable point of the plant, for example in place of the demineralised water;
b) in another case, the whole stream 28 is sent to decomposition to recover the organic compounds in form of NH₃ and CO₂, obtaining an aqueous stream (stream 29) substantially void of impurities, which can be discharged or re-used in a suitable point of the plant, and an aqueous stream 30 to recycle to the quench Q (through the Abs absorber).

The following examples of embodiment are provided to illustrate the present invention, not-limitative of the scope of protection of the annexed claims.

### EXAMPLE 1

A moulding compound has been prepared with the following recipe according to the state of the art:

| | |
|---|---|
| base mixture | 97.09 % by weight |
| zinc stearate | 0.97 % by weight |
| catalyst | 0.49 % by weight |
| titanium dioxide | 1.46 % by weight |

The base mixture is composed of alpha-cellulose and melamine-formaldehyde resin (in this case, the cellulose/resin weight ratio in the mixture was 30/70, and the formaldehyde/melamine molar ratio in the resin was 2/1) ;
the melamine-formaldehyde resin has been obtained from melamine coming from a plant fed with urea, said urea not containing formaldehyde;
the zinc stearate is a fluidizing and lubricating agent;
the catalyst is a reticulation catalyst;
the titanium dioxide is a whitening, matting and coating agent, granting that the product does not have a translucent aspect (tending to the porcelain colour), but that is white and bright (if no dyestuffs are added as in the present example 1) or coloured and bright (in the case in which dyestuffs are present).

### EXAMPLE 2

A moulding compound has been prepared with the following recipe according to the present invention:

| | |
|---|---|
| base mixture | 98.04 % by weight |
| zinc stearate | 0.98 % by weight |
| catalyst | 0.49 % by weight |
| titanium dioxide | 0.49 % by weight |

The base mixture had the same composition of Example 1; the melamine-formaldehyde resin has been obtained from melamine coming from a plant fed with urea containing 250 ppm by weight of formaldehyde.
The products obtained through the recipe of the present Example 2, carried out always in the absence of dyestuff, are white and are characterized by a greater whiteness and brightness with respect to the products obtained through the recipe of Example 1, although it has been used about 1/3 of titanium dioxide used in the Example 1.

## Claims

1. Use of urea containing formaldehyde in a process for the production of melamine by the pyrolysis of urea.

2. Use according to claim 1, **characterized in that** the formaldehyde is present in the urea in an amount ranging from 10 to 5,000 ppm by weight.

3. Use according to claim 1, **characterized in that** the formaldehyde is present in the urea in an amount ranging from 50 to 2,000 ppm by weight.

4. Use according to claim 1, **characterized in that** the formaldehyde is present in the urea in an amount ranging from 100 to 500 ppm by weight.

5. Use according to any of the claims 1-4, **characterized in that** the formaldehyde is present in the urea following the same process for the production of urea.

6. Use according to any of the claims 1-5, **characterized in that** the formaldehyde is added to the urea coming from urea plants before the feeding of said urea to the melamine plant.

7. Use according to any of the claims 1-6, **characterized in that** the urea is any type of urea or a mixture of various types of urea.

8. Use according to any of the claims 1-7, **characterized in that** the formaldehyde is added as such or as a compound or mixture containing formaldehyde.

9. A process for the production of melamine by the pyrolysis of urea, said process being **characterized in that** it is effected starting from urea containing formaldehyde.

10. The process according to claim 9, **characterized in that** it is a high-pressure process or a low-pressure process.

11. The process according to any of the claims 9-10, **characterized in that** the formaldehyde is present in the urea in an amount ranging from 10 to 5,000 ppm by weight.

12. The process according to any of the claims 9-11, **characterized in that** the formaldehyde is present in the urea following the same process for the production of urea.

13. The process according to any of the claims 9-12, **characterized in that** the formaldehyde is added to the urea coming from urea plants before the feeding of said urea to the melamine plant.

14. The process according to any of the claims 9-13, **characterized in that** the urea is any type of urea or a mixture of various types of urea.

15. The process according to any of the claims 9-14, **characterized in that** the formaldehyde is added as such or as a compound or mixture containing formaldehyde.

## Patentansprüche

1. Verwendung von Formaldehyd enthaltendem Harnstoff in einem Verfahren zur Herstellung von Melamin durch Pyrolyse von Harnstoff.

2. Verwendung nach Anspruch 1, **dadurch gekennzeichnet, dass** der Formaldehyd im Harnstoff in einer Menge im Bereich von 10 bis 5000 Gewichts-ppm vorkommt.

3. Verwendung nach Anspruch 1, **dadurch gekennzeichnet, dass** der Formaldehyd im Harnstoff in einer Menge im Bereich von 50 bis 2000 Gewichts-ppm vorkommt.

4. Verwendung nach Anspruch 1, **dadurch gekennzeichnet, dass** der Formaldehyd im Harnstoff in einer Menge im Bereich von 100 bis 500 Gewichts-ppm vorkommt.

5. Verwendung nach einem der Ansprüche 1-4, **dadurch gekennzeichnet, dass** der Formaldehyd im Harnstoff in Folge desselben Verfahrens zur Herstellung von Harnstoff vorkommt.

6. Verwendung nach einem der Ansprüche 1-5, **dadurch gekennzeichnet, dass** der Formaldehyd dem Harnstoff, der aus Harnstoffproduktionsanlagen kommt, vor der Einspeisung des Harnstoffs in die Melaminproduktionsanlage zugefügt wird.

7. Verwendung nach einem der Ansprüche 1-6, **dadurch gekennzeichnet, dass** der Harnstoff jeder beliebige Harnstofftyp ist oder ein Gemisch verschiedener Harnstofftypen.

8. Verwendung nach einem der Ansprüche 1-7, **dadurch gekennzeichnet, dass** der Formaldehyd als solcher zugefügt wird oder als Verbindung oder als Formaldehyd enthaltendes Gemisch.

9. Verfahren zur Herstellung von Melamin durch Pyrolyse von Harnstoff, wobei das Verfahren **dadurch gekennzeichnet ist, dass** es ausgehend von Formaldehyd enthaltendem Harnstoff durchgeführt wird.

10. Verfahren nach Anspruch 9, **dadurch gekennzeichnet, dass** es ein Hochdruckverfahren oder ein Niedrigdruckverfahren ist.

11. Verfahren nach einem der Ansprüche 9-10, **dadurch gekennzeichnet, dass** der Formaldehyd im Harnstoff in einer Menge im Bereich von 10 bis 5000 Gewichts-ppm vorkommt.

12. Verfahren nach einem der Ansprüche 9-11, **dadurch gekennzeichnet, dass** der Formaldehyd im Harnstoff in Folge desselben Verfahrens zur Herstellung von Harnstoff vorkommt.

13. Verfahren nach einem der Ansprüche 9-12, **dadurch gekennzeichnet, dass** der Formaldehyd dem Harnstoff, der aus Harnstoffproduktionsanlagen kommt, vor der Einspeisung des Harnstoffs in die Melaminproduktionsanlage zugefügt wird.

14. Verfahren nach einem der Ansprüche 9-13, **dadurch gekennzeichnet, dass** der Harnstoff jeder beliebige Harnstofftyp ist oder ein Gemisch verschiedener Harnstofftypen.

15. Verfahren nach einem der Ansprüche 9-14, **dadurch gekennzeichnet, dass** der Formaldehyd als solcher zugefügt wird oder als Verbindung oder als Formaldehyd enthaltendes Gemisch.

## Revendications

1. Utilisation d'urée contenant du formaldéhyde dans un procédé de production de mélamine par la pyrolyse d'urée.

2. Utilisation selon la revendication 1, **caractérisée en ce que** le formaldéhyde est présent dans l'urée dans une quantité allant de 10 à 5 000 ppm en poids.

3. Utilisation selon la revendication 1, **caractérisée en ce que** le formaldéhyde est présent dans l'urée dans une quantité allant de 50 à 2 000 ppm en poids.

4. Utilisation selon la revendication 1, **caractérisée en ce que** le formaldéhyde est présent dans l'urée dans une quantité allant de 100 à 500 ppm en poids.

5. Utilisation selon l'une quelconque des revendications 1 à 4, **caractérisée en ce que** le formaldéhyde est présent dans l'urée en suivant le même procédé de production d'urée.

6. Utilisation selon l'une quelconque des revendications 1 à 5, **caractérisée en ce que** le formaldéhyde est ajouté à l'urée provenant d'usines à urée avant l'alimentation de l'usine à mélamine en ladite urée.

7. Utilisation selon l'une quelconque des revendications 1 à 6, **caractérisée en ce que** l'urée est tout type d'urée ou un mélange de divers types d'urée.

8. Utilisation selon l'une quelconque des revendications 1 à 7, **caractérisée en ce que** le formaldéhyde est ajouté tel quel ou sous forme de composé ou de mélange contenant du formaldéhyde.

9. Procédé de production de mélamine par la pyrolyse d'urée, ledit procédé étant **caractérisé en ce qu'**il est effectué en démarrant à partir d'urée contenant du formaldéhyde.

10. Procédé selon la revendication 9, **caractérisé en ce qu'**il s'agit d'un procédé à haute pression ou d'un procédé à basse pression.

11. Procédé selon l'une quelconque des revendications 9 à 10, **caractérisé en ce que** le formaldéhyde est présent dans l'urée dans une quantité allant de 10 à 5 000 ppm en poids.

12. Procédé selon l'une quelconque des revendications 9 à 11, **caractérisé en ce que** le formaldéhyde est présent dans l'urée en suivant le même procédé de production de l'urée.

13. Procédé selon l'une quelconque des revendications 9 à 12, **caractérisé en ce que** le formaldéhyde est ajouté à l'urée provenant d'usines à urée avant l'alimentation de l'usine à mélamine en ladite urée.

14. Procédé selon l'une quelconque des revendications 9 à 13, **caractérisé en ce que** l'urée est tout type d'urée ou un mélange de divers types d'urée.

15. Procédé selon l'une quelconque des revendications 9 à 14, **caractérisé en ce que** le formaldéhyde est ajouté tel quel ou sous forme de composé ou de mélange contenant du formaldéhyde.
